# FASCICULE DE BREVET EUROPEEN

(11) **EP 3 206 477 B1**
(45) Date de publication et mention de la délivrance du brevet: **12.08.2020**
(21) Numéro de dépôt: 15778346.5
(22) Date de dépôt: 13.10.2015
(51) Int. Cl.: A01G 33/00, C12N 1/12, A61K 8/73, A61K 47/36, A61K 31/715

(54) **PROCEDE DE DEMUCILAGINATION**
ENTBASTUNGSVERFAHREN
DEGUMMING METHOD

(30) Priorité: 13.10.2014 FR 1459796
(43) Date de publication de la demande: 23.08.2017
(73) Titulaire: Algosource, 44600 Saint Nazaire (FR); Université de Nantes, 44035 Nantes Cedex 1 (FR)
(72) Inventeur: PRUVOST, Jérémy, F-44250 Saint Brevin Les Pins (FR); JAOUEN, Pascal, F-445120 Le Pouliguen (FR); MARCHAL, Luc, F-44600 Saint Nazaire (FR); JUBEAU, Sébastien, F-44350 Guerande (FR); FLEURENCE, Joël, 44700 Orvault (FR)
(74) Mandataire: Nony
(86) Numéro de dépôt international: PCT/EP2015/073689
(87) Numéro de publication internationale: WO 2016/059058

(56) Documents cités:
- EP-A2- 0 311 496
- PATEL ANIL KUMAR ET AL: "Separation and fractionation of exopolysaccharides fromPorphyridium cruentum", BIORESOURCE TECHNOLOGY, vol. 145, 20 décembre 2012 (2012-12-20), pages 345-350, XP028706121, ISSN: 0960-8524, DOI: 10.1016/J.BIORTECH.2012.12.038
- MARCATI ALAIN ET AL: "Extraction and fractionation of polysaccharides and B-phycoerythrin from the microalga Porphyridium cruentum by membrane technology", ALGAL RESEARCH-BIOMASS BIOFUELS AND BIOPRODUCTS, vol. 5, juillet 2014 (2014-07), pages 258-263, XP002739397, ISSN: 2211-9264
- KADAM S U ET AL: "Application of novel extraction technologies for bioactives from marine algae", JOURNAL OF AGRICULTURAL AND FOOD CHEMISTRY 20130522 AMERICAN CHEMICAL SOCIETY USA, vol. 61, no. 20, 22 mai 2013 (2013-05-22), pages 4667-4675, XP002739398, DOI: 10.1021/JF400819P
- DOUCHA J ET AL: "Influence of processing parameters on disintegration of Chlorella cells in various types of homogenizers", APPLIED MICROBIOLOGY AND BIOTECHNOLOGY, SPRINGER, BERLIN, DE, vol. 81, no. 3, 29 août 2008 (2008-08-29), pages 431-440, XP019654147, ISSN: 1432-0614, DOI: 10.1007/S00253-008-1660-6
- HONGLI ZHENG ET AL: "Disruption ofCells for the Release of Biodiesel-Producing Lipids: A Comparison of Grinding, Ultrasonication, Bead Milling, Enzymatic Lysis, and Microwaves", APPLIED BIOCHEMISTRY AND BIOTECHNOLOGY ; PART A: ENZYME ENGINEERING AND BIOTECHNOLOGY, HUMANA PRESS INC, NEW YORK, vol. 164, no. 7, 24 février 2011 (2011-02-24), pages 1215-1224, XP019918055, ISSN: 1559-0291, DOI: 10.1007/S12010-011-9207-1
- LI ZHANG ET AL: 31 octobre 2013 (2013-10-31), ULTRASONICS SONOCHEMISTRY ELSEVIER B.V. NETHERLANDS, VOL. 21, NR. 2, PAGE(S) 576 - 581, XP002739399, ISSN: 1350-4177 abrégé 2.2. High-intensity ultrasound treatment; page 577 page 576, colonne de gauche, ligne 13 - ligne 18

## Description

La présente invention se rapporte au domaine de la valorisation de la biomasse notamment de la biomasse algale, plus précisément la présente invention concerne un procédé d'extraction de polysaccharides issus de microorganismes notamment de cyanobactéries, micro-algues, bactéries, micro-organismes photosynthétiques ainsi que de champignons filamenteux,

Les micro-algues sont des organismes eucaryotes, la plupart du temps unicellulaires, délimitées par une membrane plasmique et une paroi. La composition et la structure de cette paroi peuvent être variables selon la microalgue considérée.

Ainsi, chez certaines microalgues vertes telles que *Chlorella,* elle est constituée de cellulose et possède une forte rigidité entraînant une résistance élevée de l'algue vis-à-vis des contraintes mécaniques.

Pour les micro-algues appartenant à la classe des diatomées, la paroi, aussi appelée frustule, est constituée de silice cristallisée. Cette dernière est plus cassante que celle de *Chlorella.*

Enfin, d'autres espèces mettent en place autour de leur cellule, une gangue polysaccharidique pour se protéger des agressions environnementales. L'épaisseur de cette gangue varie au cours du temps, elle est assez fine lors de la croissance exponentielle de la micro-algue puis plus épaisse en phase stationnaire. En phase stationnaire, le taux de production de polysaccharides membranaires est supérieur au taux de solubilisation dans le milieu de culture.

Dans le but de valoriser la totalité de la biomasse micro-algale, dans un objectif de bioraffinage, il est nécessaire de fractionner, d'isoler les différents métabolites de ladite micro-algue qui sont généralement solubles dans le milieu de culture.

Ainsi les principaux métabolites sont les polysaccharides, les protéines, et les pigments.

Les microorganismes plus particulièrement visés par l'invention comprennent plusieurs sortes de polysaccharides : les polysaccharides intracellulaires et les polysaccharides extracellulaires nommés polysaccharides membranaires.

Les microorganismes plus particulièrement visés par l'invention sont, de préférence, unicellulaires.

Or, pour ces polysaccharides, se pose le problème du rendement d'extraction. En effet, lors de la culture des micro-algues, une partie des polysaccharides membranaires se trouve libre, solubilisée dans le milieu. Cette fraction libre peut être aisément récupérée. Cependant, il existe également des polysaccharides membranaires liés, c'est-à-dire fixés à l'extérieur des cellules, sur leur périphérie. Ces polysaccharides membranaires liés sont parfois nommés exopolysaccharidse ou EPS.

Chez certaines espèces comme *Porphyridium cruentum,* on estime que les polysaccharides membranaires liés constituent environ 50 % de la totalité des polysaccharides membranaires.

Le problème posé à l'origine de la présente demande réside dans la difficulté d'extraire de manière sélective les polysaccharides membranaires, c'est-à-dire d'extraire non seulement les polysaccharides membranaires libres mais aussi de libérer les polysaccharides membranaires liés dans le milieu sans provoquer de lyse cellulaire.

Plus particulièrement, le problème posé à l'origine de la présente demande réside dans la difficulté d'extraire les polysaccharides membranaires liés dans le milieu sans provoquer de lyse cellulaire.

En effet, la lyse cellulaire conduit à libérer dans le milieu tout ou partie des constituants de la cellule, ce qui conduit à complexifier la purification des différents métabolites et notamment des polysaccharides membranaires.

Il est connu d'extraire des polysaccharides de la biomasse, notamment des algues, par exemple US 4,960,697 divulgue un procédé pour extraire les polysaccharides solubilisés, notamment les glucanes non ioniques mettant en œuvre un cation divalent puis un solvant organique miscible à l'eau afin de faire précipiter les polysaccharides.

EP 0311 496 décrit un procédé de production et d'extraction, par voie chimique, des polysaccharides péri- et exocellulaires mettant en œuvre une étape de précipitation des polysaccharides en particulier avec du cétyl-pyridium.

WO 83/03738 décrit un procédé de culture de micro-algue de souche *Porphyridium cruentum* et un procédé d'extraction des polysaccharides membranaires mettant en œuvre des étapes de basification de la solution au moyen d'une solution de soude concentrée, de chauffage, d'ajout à froid d'une solution d'acide chlorhydrique. Cette hydrolyse est suivie d'une précipitation des polysaccharides au moyen d'un solvant organique notamment en ajoutant de 2 à 3 volumes d'éthanol.

Ce procédé permet l'extraction des polysaccharides intra et extracellulaires.

Ces procédés entrainent cependant une destruction de la biomasse qui n'est pas compatible avec l'objectif de valorisation des différents métabolites.

Le procédé selon l'invention permet l'extraction des polysaccharides membranaires liés.

Le procédé selon l'invention permet ainsi l'extraction des polysaccharides membranaires selon un rendement élevé avec un taux réduit de lyse cellulaire.

De manière particulière, l'invention vise l'extraction de polysaccharides issus de micro-algues, cyanobactéries, bactéries, champignons filamenteux, micro-organismes photo synthétiques.

Un premier objet de la présente invention vise un procédé d'obtention de polysaccharides membranaires à partir d'un organisme, ledit procédé comprenant au moins une étape d'extraction desdits polysaccharides membranaires accompagnée d'une extraction réduite des protéines solubles, par traitement mécanique dudit organisme dans un broyeur à billes ou par traitement physique dudit organisme unicellulaire à l'aide d'ultrasons, dans lequel :
- le procédé est réalisé par traitement mécanique avec un broyeur à billes avec un taux de remplissage allant de 50% à 80% volume/volume, à une température allant de 18 à 40°C, le diamètre moyen des billes (d_{GM}) variant de 1,5 10⁻³ à 2,5 10⁻³ m et la vitesse d'agitation en bout de pale variant de 5 à 20 m.sec⁻¹,
   ou
- le procédé est réalisé par traitement physique dudit organisme à l'aide d'ultrasons dans les conditions suivantes :
   ∘ l'énergie varie de 500 à 5000 J,
   ∘ la puissance varie de 200 à 400 W, et
   ∘ le débit d'alimentation du réacteur varie de 0,150 L/min à 3 L/min

Plus particulièrement, ce procédé vise l'obtention des polysaccharides membranaires liés.

Les organismes visés sont plus particulièrement choisis parmi les microorganismes, les organismes unicellulaires et les champignons filamenteux.

Ledit traitement mécanique ou physique peut être mis en œuvre par toute autre technique permettant de générer un stress mécanique suffisamment contrôlé sur la cellule. On peut citer en particulier la mise en cavitation dans une conduite qui consiste à générer une dépression suffisante dans la culture en circulation pour créer l'apparition de bulles générant une onde de vibration ayant l'effet mécanique escompté.

Un deuxième objet décrit vise les utilisations de la fraction des polysaccharides solubles obtenue.

De manière plus détaillée, il est décrit un procédé d'obtention des polysaccharides membranaires comprenant les étapes suivantes, dans cet ordre :
- disposer d'un milieu comprenant au moins un organisme choisi parmi les microorganismes, les organismes unicellulaires et les champignons filamenteux,
- effectuer sur ledit milieu une étape d'extraction desdits polysaccharides membranaires accompagnée d'une extraction réduite des protéines solubles, par traitement mécanique dudit organisme dans un broyeur à billes ou par traitement physique dudit organisme unicellulaire à l'aide d'ultrasons,
- extraire la fraction des polysaccharides solubles dudit milieu,
- éventuellement purifier ladite fraction comprenant les polysaccharides.

Ce procédé permet l'extraction des polysaccharides membranaires liés.

Avantageusement, l'étape d'extraction desdits polysaccharides membranaires n'est pas accompagnée d'une extraction des protéines solubles.

De préférence, ce procédé est mis en œuvre en mode circuit.

Par « extraction réduite des protéines solubles » au sens de la présente invention, on entend que la sélectivité de l'extraction, définie comme le ratio du rendement d'extraction des protéines sur le rendement d'extraction des EPS, est inférieure à 0,7.

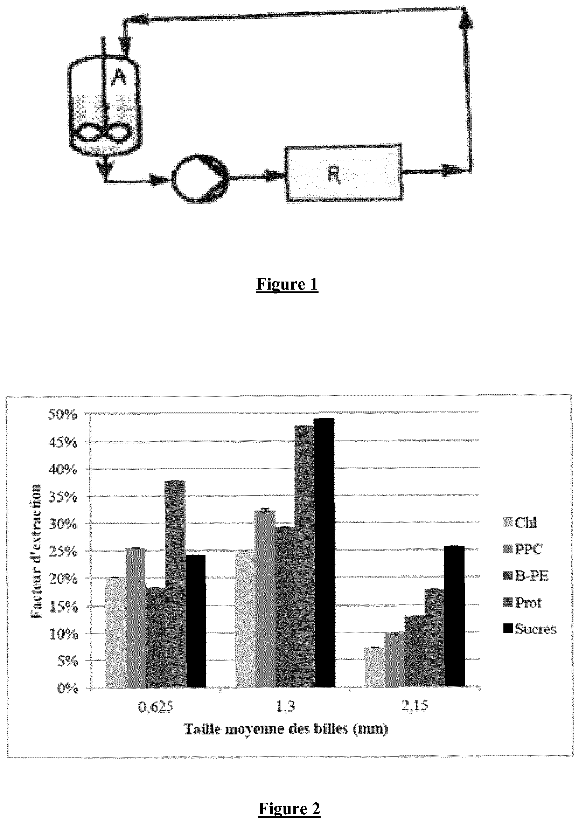

Le principe du « mode circuit » utilisé selon la présente invention, est présenté à la Figure 1.

Le mode circuit permet de traiter un volume en « *batch* » sur un dispositif continu de manière simple.

Selon ce mode, le milieu à traiter est placé dans le récipient d'alimentation (A) où il est soumis à une agitation puis envoyé dans le réacteur (R) où il est soumis à un traitement apte à augmenter la concentration en polysaccharides solubles dans ce milieu sans augmenter de manière aussi élevée la concentration en protéines solubles de ce milieu. A la fin du traitement, le milieu est renvoyé vers le récipient d'alimentation où il est soumis à une agitation. Le milieu à traiter est introduit N fois dans le réacteur pour y être traité, ce qui correspond au nombre de passages.

Le dosage des différents métabolites produits lors du traitement a lieu dans le récipient d'alimentation soit à la fin des N traitements, soit après chaque passage.

Dans le cas où le dosage des différents métabolites est effectué après chaque passage, tant que la concentration en polysaccharides du milieu réactionnel augmente, ledit milieu est renvoyé dans le réacteur. Le nombre de passages dans le réacteur est généralement compris entre 1 et 10, bornes incluses.

Le temps de séjour da la composition correspond à l'ensemble des passages de la composition dans le réacteur.

Le procédé selon l'invention permet d'éroder les cellules et par conséquent de décrocher la fraction des polysaccharides membranaires liés (ou EPS) sans altérer de manière significative l'intégrité des cellules.

Ce traitement est également nommé une démucilagination.

Le procédé selon la présente invention permet, d'une part, d'augmenter le rendement d'extraction des polysaccharides par rapport aux procédés de l'art antérieur et d'autre part de faciliter les opérations subséquentes de purification des polysaccharides.

Le rendement d'extraction des polysaccharides a, en effet, pu être augmenté de manière à extraire jusqu'à 90 % en poids de polysaccharides membranaires par rapport au poids total des polysaccharides membranaires.

En outre, comme déjà mentionné, le procédé selon l'invention, dans la mesure où il n'altère que modérément l'intégrité cellulaire, permet d'éviter que d'autres constituants hydrosolubles de la cellule, par exemple les protéines hydrosolubles, les chlorophylles soient libérés en trop grande quantité dans le milieu. Il présente ainsi l'avantage de conduire à un produit de réaction moins visqueux que les produits obtenus par les procédés de l'art antérieur conduisant à une lyse cellulaire.

En effet dans l'optique de bioraffinage de la biomasse, une extraction sélective de chacun des constituants des cellules est recherchée. Or, les extractions sélectives peuvent être perturbées par la présence de certains composés tels que notamment les polysaccharides membranaires qui ont tendance à se complexer avec d'autres molécules du milieu tels que les protéines.

Ainsi, le procédé selon l'invention permet d'augmenter le rendement de récupération et la pureté des polysaccharides hydrosolubles dans le milieu en fin de réaction.

Les opérations de purification des composants sont facilitées notamment à cause de la proportion réduite des protéines dans le milieu récupéré contenant les polysaccharides, ou encore à cause du retrait des polysaccharides des cellules facilitant ainsi les étapes aval de récupération d'autres composés cellulaires susceptibles de se complexifier avec les polysaccharides.

Le procédé selon l'invention doit être considéré comme permettant une récupération ciblée et quasi-totale des polysaccharides membranaires libres et liés.

Un autre avantage du procédé selon l'invention est qu'il peut être mis en œuvre directement sur le milieu de culture des organismes notamment unicellulaires, en particulier sur une suspension de micro-algues en sortie de production, ce qui contribue à réduire les volumes d'eau utilisés pour mettre en œuvre ledit procédé.

En outre, ce procédé permet également d'éviter la mise en œuvre d'une étape de séchage, étape par définition longue et coûteuse, tant en énergie que financièrement.

Selon une variante du procédé, l'étape de traitement mécanique ou physique est suivie d'une étape de mesure de la quantité de polysaccharides membranaires extraits.

Le procédé selon l'invention peut comprendre une pluralité d'étapes de traitement, avantageusement de 2 à 10 étapes de traitement.

Le procédé peut également comprendre au moins une étape de traitement, mécanique ou physique, suivie d'une étape de mesure de la quantité des polysaccharides membranaires extraits.

Toute méthode permettant de déterminer la quantité des polysaccharides membranaires extraits peut être mise en œuvre.

Ces méthodes relèvent des compétences générales de l'homme du métier telles que les méthodes colorimétriques.

En particulier, le dosage des polysaccharides est réalisé par la méthode colorimétrique utilisant du phénol et de l'acide sulfurique (Dubois M, Gilles KA, Hamilton JK, Rebers PA, Smith F. Colorimetric method for détermination of sugars and related substances. Anal. Chem. 1956;28:350-356).

Selon une autre variante, le procédé selon l'invention peut également comprendre une pluralité d'étapes de traitement, avec au moins deux étapes de traitement qui sont suivies chacune d'une étape de mesure de la quantité de polysaccharides membranaires qui ont été extraits.

En particulier ledit procédé peut être interrompu lorsque la quantité de polysaccharides membranaires mesurée à l'issue d'une étape N de traitement est sensiblement identique à la quantité de polysaccharides membranaires mesurée à l'issue d'une étape N-X de traitement. X étant un nombre de traitements inférieur au nombre total de traitements N, en particulier X est égal à 1.

Avantageusement le procédé selon l'invention comprend une étape consistant à extraire la fraction des polysaccharides solubles dudit milieu.

Avantageusement l'étape d'extraction des polysaccharides membranaires est suivie d'une étape de purification desdits polysaccharides.

Dans le procédé selon l'invention, l'étape d'extraction des polysaccharides membranaires peut être suivie d'une étape de purification desdits polysaccharides.

Il relève des compétences de l'homme du métier de choisir le procédé de purification adapté, classiquement ce procédé sera choisi par me les méthodes par changement de pH, les méthodes par précipitation à l'éthanol ; les séparations physiques à l'aide d'une membrane.

Le procédé d'extraction de polysaccharides selon l'invention peut être mis en œuvre à partir de tout organisme, de préférence microorganisme, de manière préférée, il est mis en œuvre à partir de cyanobactéries, micro-algues, bactéries, champignons filamenteux.

Avantageusement, le procédé selon l'invention est mis en œuvre lors de la phase stationnaire de la microalgue c'est-à-dire dans un état physiologique intermédiaire ou mature, à la fin ou après la phase dite de croissance exponentielle.

De préférence l'organisme est choisi parmi : la division des Rhodophytes et plus spécifiquement parmi les genres Porphyridium et Rhodella, la Spiruline et la Dunaliella, et de préférence parmi les souches suivantes *Porphyridium cruentum, Arthospira platensis, Botryococcus braunii, Cryptecodinium conhii, Chlorella autotrophica, Navicula incerta et Rhodosorusmarinus.*

### Procédé mettant en œuvre un broyeur à billes

Selon une première variante préférée, le procédé selon l'invention comprend une étape lors de laquelle est utilisé un broyeur à billes.

Les broyeurs à billes sont classiquement utilisés pour l'homogénéisation des produits visqueux tels que les peintures et aussi pour broyer les minéraux. Les broyeurs à billes comprennent une enceinte, par exemple un bol fermé par un couvercle, destinée à recevoir la composition à traiter, ladite enceinte étant alimentée *via* une pompe en composition à traiter.

Classiquement le taux de remplissage, correspondant au pourcentage du volume du bol occupé par les billes, du broyeur à billes va de 50 % à 80 %, de préférence de 65 % à 80%.

Le contenu de l'enceinte, hors billes, comprend essentiellement l'organisme unicellulaire ou la suspension à traiter.

Le taux de remplissage pourra être adapté notamment en fonction de la nature des billes utilisées. En effet dans certains cas, une agglomération des billes entre les pâles de l'agitateur a pu être observée.

Dans le cas de billes, notamment en verre, le taux de remplissage en billes du broyeur varie avantageusement de 60 % à 80 % et de préférence de 65 % à 80 % volume/volume.

Il relève des compétences de l'homme du métier de sélectionner le taux de remplissage adapté au milieu à traiter.

Le débit d'alimentation de la composition dans le broyeur va généralement de 150 ml/min à 200 ml/min. Il relève également des compétences de l'homme du métier de sélectionner le débit d'alimentation adapté au milieu à traiter.

Le traitement au broyeur à billes est généralement effectué à une température comprise allant de 18 à 40 °C, de préférence allant de 18 à 25 °C.

Selon une première variante du procédé, le traitement mécanique utilisé dans le procédé selon l'invention met en œuvre un broyeur à billes en verre dans les conditions suivantes :
- le diamètre moyen des billes (d_{GM}) varie de 1,5 10⁻³ à 2,5 10⁻³ m,
- la vitesse d'agitation en bout de pale (v) varie de 5 à 20 m.sec⁻¹.

A l'issue de l'étape de broyage à billes, la composition traitée est récupérée puis les polysaccharides présents dans ladite composition sont extraits et éventuellement purifiés.

Selon une mise en œuvre du procédé, une seule étape de broyage à billes est effectuée.

Selon une mise en œuvre préférée du procédé décrit, le traitement dans un broyeur à billes est répété au moins deux fois, de préférence entre deux et dix fois et avantageusement entre trois et quatre fois.

De préférence, le traitement dans un broyeur à billes est réalisé pendant un temps de séjour allant de 2 à 50 minutes, de préférence de 10 à 40 minutes.

### Procédé mettant en œuvre des ultrasons

Selon ce procédé décrit, le milieu à traiter est pompé à travers le réacteur ultrasons au sein duquel il va être soumis au traitement ultrasons.

Selon une deuxième variante du procédé, le traitement physique met en œuvre un réacteur ultrasons dans les conditions suivantes :
- l'énergie varie de 500 à 5 000 J,
- la puissance varie de 200 à 400 W,
- le débit d'alimentation du réacteur varie de 0,150 L/min à 3 L/min.

A l'issue de l'étape de traitement aux ultrasons, la composition traitée est récupérée puis les polysaccharides présents dans ladite composition sont extraits et éventuellement purifiés.

De préférence, le traitement mécanique aux ultrasons est réalisé pendant une durée variant de 5 à 30 secondes.

### Propriétés des polysaccharides membranaires

La présente description vise encore les utilisations de la fraction des polysaccharides solubles obtenue suivant le procédé selon l'invention, ledit procédé comprenant une étape consistant à extraire la fraction des polysaccharides solubles dudit milieu éventuellement suivie d'une étape de purification desdits polysaccharides.

Ladite fraction des polysaccharides membranaires est utilisée dans des compositions chimiques, alimentaires, cosmétiques ou pharmaceutiques.

Ladite fraction des polysaccharides membranaires présente des propriétés intéressantes notamment des propriétés texturantes.

Elle peut être utilisée en tant qu'agents épaississants, agents gélifiants, tensioactifs ou encore stabilisateurs des compositions et plus particulièrement des émulsions.

Elle peut ainsi être utilisée dans des produits phyto-sanitaires ou en tant que produits phyto-sanitaires.

Ladite fraction des polysaccharides membranaires peut aussi être utilisée en tant que matériaux notamment des colles.

Les polysaccharides membranaires obtenus selon l'invention peuvent aussi être fractionnés en oligosaccharides.

Les oligosaccharides ainsi obtenus sont avantageusement utilisés tant que réactifs en chimie fine.

### Description des figures

La Figure 1 présente le principe du mode circuit détaillé plus haut.
La Figure 2 représente le facteur d'extraction en fonction de la taille moyenne des billes.
Sur la Figure 3 sont représentées, d'une part, les courbes des facteurs de solubilisation des protéines et des polysaccharides et, d'autre part, l'indice de pureté (IP) des sucres, en fonction du diamètre moyen des billes.
La Figure 4 représente le facteur d'extraction aux ultrasons sur une biomasse jeune en fonction du nombre de passes à 2 000 J (200 W).
La Figure 5 représente le facteur d'extraction aux ultrasons sur une biomasse d'âge intermédiaire en fonction du nombre de passes à 2 000 J (200 W).
La Figure 6 représente le facteur d'extraction aux ultrasons sur une biomasse mature en fonction du nombre de passes à 2 000 J (400 W).
La Figure 7 représente le facteur d'extraction aux ultrasons sur une biomasse mature en fonction du nombre de passes à 2 000 J (200 W).

Les exemples qui suivent illustrent l'invention sans en limiter la portée.

### Exemples

Pour tous ces exemples, on utilise un milieu de culture de *Porphyridium cruentum.* Cette biomasse a été cultivée en photobioréacteur tubulaire de 10 L.

Les protéines (notées « prot » sur les figures) sont mesurées par absorbance à 280 nm, mesure éventuellement complétée d'un dosage des protéines réalisé selon le protocole BCA afin de vérifier la justesse des mesures spectro-photométriques.

La quantification des pigments : chlorophylles totales et caroténoïdes totaux (notées respectivement Chl et PPC sur les figures) est réalisée par spectrométrie respectivement à 678 nm et 416 nm.

La B-Phycoérythrine (notée B-PE sur les figures) est dosée par le protocole de Bermejo.

### 1. Procédé utilisant un broyeur à billes

### 1.1 Exemple : détermination du facteur d'extraction en fonction du diamètre moyen des billes

Le milieu de culture de *Porphyridium cruentum* a été traité au moyen d'un broyeur à billes (DynoMill Mutlilab, WAB, Suisse) en verre dans les conditions suivantes :
Q : débit d'alimentation en ml/min : 170
ϕ : Taux de remplissage du broyeur : 75 %
n : vitesse d'agitation : 2 389 min⁻¹ →ν : vitesse en bout de pale des agitateurs 8 m.sec⁻¹.

A titre de référence, un broyage Haute Pression (2 700b) permettant la destruction cellulaire et par conséquent la libération de la totalité (100 %) des métabolites en solution/suspension stable donc de la totalité des polysaccharides est réalisé. L'unité de mesure pour chaque métabolite sera le facteur d'extraction dont le maximum (100%) correspond à la réponse obtenue avec le broyat HP.

Après l(es) étape(s) de broyage, une centrifugation est effectuée (13 400x g, 10 min), les analyses sont effectuées sur le surnageant constituant l'extrait aqueux de microalgues.

Les résultats sont présentés en Figure 2.

Trois traitements de broyage ont été effectués avec des billes de diamètre moyen différent :
a) avec des billes de diamètre moyen de 0,625 10⁻³ m, on observe une déconstruction des cellules, la sélectivité des protéines est maximale. Les billes ne sont pas adaptées à la récupération des polysaccharides. Lors de la déconstruction des cellules, la compartimentation cellulaire est respectée, la libération des métabolites est généralement sélective.
b) avec des billes de diamètre moyen de 1,3 10⁻³ m, on observe une destruction qui s'apparente à une désintégration cellulaire (la cellule est complètement déstructurée, les débris sont fins) qui s'accompagne d'une libération des protéines, des polysaccharides et aussi des pigments de façon homogène. La libération homogène de tous les pigments traduit une destruction profonde de la cellule, ce procédé est le moins sélectif entre protéines et polysaccharides, il ne permet pas une extraction sélective des polysaccharides.
c) les billes de diamètre moyen de 2,15 10⁻³ m sont les plus adaptées à l'extraction sélective des sucres par rapport aux métabolites cellulaires, ce procédé est celui qui conduit à la destruction cellulaire la moins importante.

### 1.2 Détermination des facteurs de solubilisation et indice de pureté

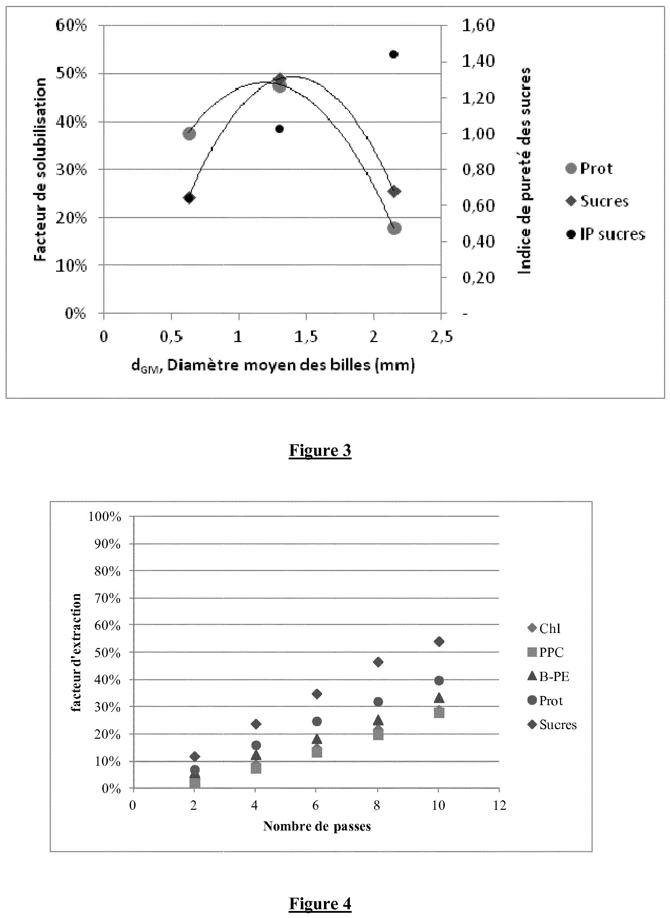

A partir des résultats obtenus à l'issue des trois broyages ci-dessus, on a tracé, en figure 3, en fonction du diamètre moyen des billes, les courbes des facteurs de solubilisation des protéines et des polysaccharides et sur le même graphe l'indice de pureté des sucres.

Il ressort de ce graphe qu'à partir d'un diamètre moyen des billes de 1,3 10⁻³ m, le facteur de solubilisation des polysaccharides est supérieur au facteur de solubilisation des protéines et que l'indice de pureté des polysaccharides augmente avec la taille des billes.

En choisissant des billes de diamètre moyen supérieur à 1,5 10⁻³ m, on se place dans des conditions où le taux de solubilisation des polysaccharides membranaires est supérieur à celui des autres composés cellulaires en particulier les protéines.

En choisissant des billes de diamètre moyen égal à 2,15 10⁻³ m, on se place dans des conditions où le taux de solubilisation des polysaccharides membranaires est faible mais où l'indice de pureté est plus élevé.

En effectuant plusieurs passages du milieu à traiter dans le broyeur à billes, avec des billes de diamètre moyen supérieur à 1,5 10⁻³ m, on parvient à obtenir des solubilisations de l'ordre de 80 %.

### 2. Procédé utilisant les ultrasons

La technologie utilisée est le Sonitube® Type SM 35/3, fourni par SYNETUDE. Il s'agit d'un tunnel à ultrason d'une puissance maximale de 400 W dont la fréquence de travail est 35 kHz

La suspension est pompée à travers le Sonitube, au sein duquel elle va être soumise au traitement ultrason (volume de 70 mL de volume de traitement). Les paramètres opératoires sont donc le débit d'alimentation ainsi que la puissance des ultrasons (200 W-400 W). En fonctionnement, une partie importante de l'énergie fournie au fluide est dissipée sous forme de chaleur. De ce fait, la température de la suspension sera suivie, afin qu'elle n'excède pas 30 °C.

Lors de chaque expérience, le broyage HP réalisé à 2 700b défini au paragraphe précédent sert de référence.

Suite à l'étape de sonication, une centrifugation est effectuée (13 000 x g, 10 min).

Toutes les analyses seront effectuées sur le surnageant, constituant l'extrait aqueux de microalgues.

Pour les ultrasons, des essais en discontinu ont été effectués.

Des dosages biochimiques de protéines ont été réalisés afin de s'affranchir du potentiel biais qu'il pourrait y avoir sur le facteur d'extraction calculé à partir de l'A₂₈₀.

Partant du constat qu'en phase stationnaire, le taux de production de polysaccharides membranaires est supérieur au taux de solubilisation dans le milieu de culture, il a été décidé de réaliser le traitement aux ultrasons sur des suspensions de *Porphyridium cruentum* dont l'état physiologique diffère.

En effet, à l'état physiologique jeune, le taux de production de polysaccharides membranaires est encore faible et en tout état de cause inférieur au taux de solubilisation dans le milieu de culture.

### a. Etat physiologique jeune

La culture était à 3,7 gMS/L dont 1,5 g/L de polysaccharides membranaires liés et 0,3 g/L de polysaccharides membranaires solubilisés. Le suivi au cours des passes successives est présenté en figure 4.

Une certaine sélectivité est observée. Le traitement aux ultrasons permet d'atteindre un relargage de plus de 50 % des polysaccharides membranaires liés, avec une relargage de protéines de l'ordre de 40 %. Néanmoins, l'état physiologique des cellules fait que la démucilagination intervient sur une quantité relativement faible des polysaccharides membranaires liés (1,5 g/L).

Cet état physiologique peut également expliquer le taux important de destruction cellulaire.

### b. Etat physiologique intermédiaire, début de phase stationnaire

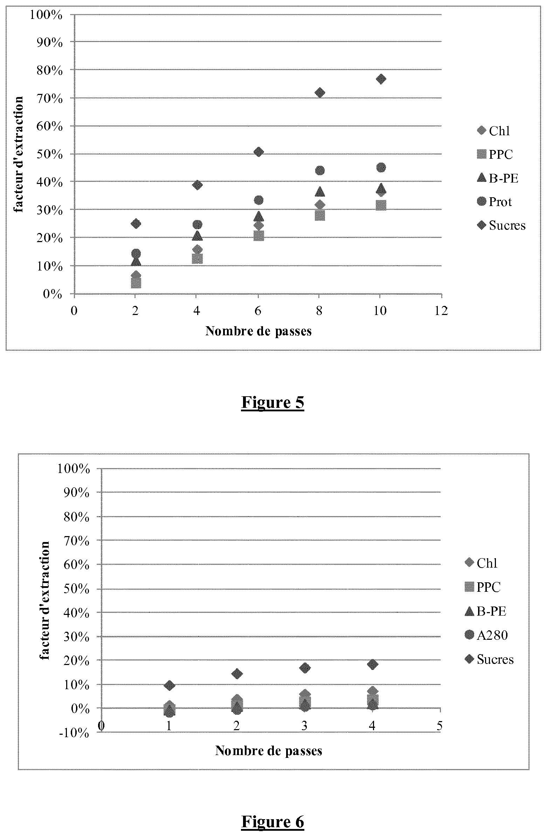

La culture était à 4,5 gMS/L dont 1,7 g/L de polysaccharides membranaires liés et 0,4 g/L de polysaccharides membranaires solubilisés. Le suivi au cours des passes successives est présenté en Figure 5.

Avec le vieillissement de la culture, la quantité d'EPS liés augmente. Le phénomène de démucilagination sélectif semble favorisé avec l'augmentation des polysaccharides membranaires liés. En effet, il est possible d'atteindre un facteur d'extraction des polysaccharides membranaires très satisfaisant (près de 80 %) en limitant le relargage de protéines solubles (< 50 %). L'épaississement de la paroi cellulaire de *Porphyridium cruentum* durant sa maturation permettrait donc d'en décrocher une plus grande partie sans toutefois lyser systématiquement les cellules.

### c. Etat physiologique mature

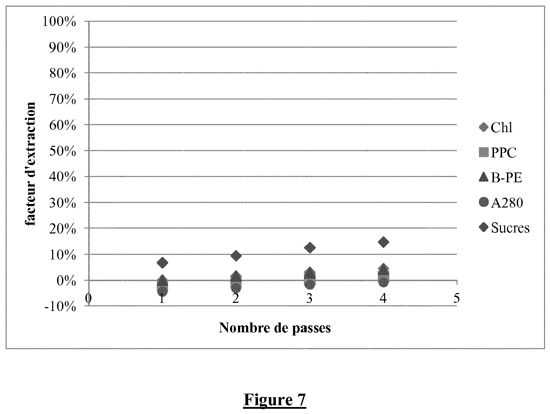

La culture était à 6,4 gMS/L dont 3,2 g/L de polysaccharides membranaires liés et 0,44 g/L de polysaccharides membranaires solubilisés. Pour cette expérience, les puissances minimales et maximales de travail ont été testées, à savoir 200 W et 400 W. Les suivis au cours des passes successives sont présentés en Figures 6 et 7.

Sur cette biomasse, une très faible lyse cellulaire est observée (< 5 %). Si la fiabilité de l'A₂₈₀ peut être contestée (quelques valeurs vers 0 %), le dosage de la B-PE est une méthode reconnue comme fiable et pourra donc servir d'indicateur de lyse (profonde).

Dans ce cadre, il semblerait que le traitement à 400 W soit plus sélectif que celui à 200 W. En effet, le rapport des facteurs d'extraction [polysaccharides (%)] / [B-PE (%)] est de 3,5 à 200 W et de 8 à 400 W. De plus, une démucilagination plus poussée est observée à 400 W (près de 20 %).

Il ressort de ces expériences que les ultrasons dans des conditions adéquates permettent la démucilagination de la micro-algue.

## Revendications

1. Procédé d'obtention de polysaccharides membranaires à partir d'un organisme choisi parmi les cyanobactéries, les micro-algues, les bactéries et les champignons filamenteux, ledit procédé comprenant au moins une étape d'extraction desdits polysaccharides membranaires accompagnée d'une extraction réduite des protéines solubles, par traitement mécanique dudit organisme dans un broyeur à billes ou par traitement physique dudit organisme à l'aide d'ultrasons, dans lequel :
- le procédé est réalisé par traitement mécanique avec un broyeur à billes avec un taux de remplissage allant de 50% à 80% volume/volume, à une température allant de 18 à 40 °C, le diamètre moyen des billes (d_{GM}) variant de 1,5 10⁻³ à 2,5 10⁻³ m et la vitesse d'agitation en bout de pale variant de 5 à 20 m.sec-1,
ou
- le procédé est réalisé par traitement physique dudit organisme à l'aide d'ultrasons dans les conditions suivantes :
- l'énergie varie de 500 à 5000 J,
- la puissance varie de 200 à 400 W, et
- le débit d'alimentation du réacteur varie de 0,150 L/min à 3 L/min

2. Procédé selon la revendication 1, le traitement mécanique dans un broyeur à billes étant réalisé pendant une durée variant de 2 à 50 minutes, de préférence de 10 à 40 minutes.

3. Procédé selon l'une des revendications 1 et 2, le taux de remplissage en billes du broyeur variant de 60 % à 80 % et de préférence de 65 % à 80 % volume/volume.

4. Procédé selon la revendication 1, le traitement aux ultrasons étant réalisé pendant une durée variant de 5 à 30 secondes.

5. Procédé selon l'une des revendications 1 à 4, l'étape de traitement mécanique ou physique étant suivie d'une étape de mesure de la quantité des polysaccharides membranaires extraits.

6. Procédé selon l'une des revendications 1 à 5, au moins une étape de traitement étant suivie d'une étape de mesure de la quantité de polysaccharides membranaires qui ont été extraits.

7. Procédé selon l'une des revendications 1 à 8, dans lequel l'organisme est choisi parmi : la division des Rhodophytes et plus spécifiquement parmi les genres Porphyridium et Rhodella, la Spiruline et la Dunaliella, et de préférence parmi les souches suivantes *Porphyridium cruentum, Arthospira platensis, Botryococcus braunii, Cryptecodinium conhii, Chlorella autotrophica, Navicula incerta et Rhodosorusmarinus.*

8. Procédé selon l'une des revendications 1 à 9 comprenant une étape consistant à extraire la fraction des polysaccharides solubles dudit milieu.

9. Procédé selon l'une des revendications 1 à 10, l'étape d'extraction des polysaccharides membranaires étant suivie d'une étape de purification desdits polysaccharides.

## Patentansprüche

1. Verfahren zur Gewinnung von Membranpolysacchariden aus einem Organismus, ausgewählt aus Cyanobakterien, Mikroalgen, Bakterien und filamentösen Pilzen, wobei das Verfahren mindestens einen Schritt der Extraktion der Membranpolysaccharide, begleitet von einer verringerten Extraktion löslicher Proteine, durch mechanische Behandlung des Organismus in eine Kugelmühle oder durch physikalische Behandlung des Organismus mithilfe von Ultraschall umfasst, wobei:
- das Verfahren durch mechanische Behandlung mit einer Kugelmühle mit einem Füllungsgrad von 50% bis 80% Volumen/Volumen bei einer Temperatur von 18 bis 40°C durchgeführt wird, wobei der mittlere Durchmesser der Kugeln (d_{GM}) von 1,5 10⁻³ bis 2,5 10⁻³ m variiert und die Rührgeschwindigkeit an der Schaufelspitze von 5 bis 20 m.s⁻¹ variiert,
oder
- das Verfahren durch physikalische Behandlung des Organismus mittels Ultraschall unter den folgenden Bedingungen durchgeführt wird:
- die Energie variiert von 500 bis 5000 J,
- die Leistung variiert von 200 bis 400 W und
- die Beschickungsrate des Reaktors variiert von 0,150 l/min bis 3 l/min.

2. Verfahren nach Anspruch 1, wobei die mechanische Behandlung in einer Kugelmühle für eine Dauer von 2 bis 50 Minuten, vorzugsweise von 10 bis 40 Minuten, durchgeführt wird.

3. Verfahren nach einem der Ansprüche 1 und 2, wobei der Kugelfüllungsgrad der Mühle von 60% bis 80% und vorzugsweise von 65% bis 80% Volumen/Volumen variiert.

4. Verfahren nach Anspruch 1, wobei die Ultraschallbehandlung für eine Dauer von 5 bis 30 Sekunden durchgeführt wird.

5. Verfahren nach einem der Ansprüche 1 bis 4, wobei auf den Schritt der mechanischen oder physikalischen Behandlung ein Schritt der Messung der Menge der extrahierten Membranpolysaccharide folgt.

6. Verfahren nach einem der Ansprüche 1 bis 5, wobei auf mindestens einen Behandlungsschritt ein Schritt der Messung der Menge der Membranpolysaccharide, die extrahiert worden sind, folgt.

7. Verfahren nach einem der Ansprüche 1 bis 8, wobei der Organismus ausgewählt ist aus: der Abteilung Rotalgen und insbesondere aus den Gattungen Porphyridium und Rhodella, Spirulina und Dunaliella und vorzugsweise aus den folgenden Stämmen *Porphyridium cruentum, Arthospira platensis, Botryococcus braunii, Cryptecodinium conhii, Chlorella autotrophica, Navicula incerta* und *Rhodosorus marinus.*

8. Verfahren nach einem der Ansprüche 1 bis 9, umfassend einen Schritt, bei dem man die Fraktion der löslichen Polysaccharide aus dem Medium extrahiert.

9. Verfahren nach einem der Ansprüche 1 bis 10, wobei auf den Schritt der Extraktion der Membranpolysaccharide ein Schritt der Reinigung der Polysaccharide folgt.

## Claims

1. A process for obtaining membrane polysaccharides from an organism selected from microorganisms, unicellular organisms and filamentous fungi, said process comprising at least one step of extracting said membrane polysaccharides, accompanied by a reduced extraction of the soluble proteins, by mechanical treatment of said organism in a ball mill or by physical treatment of said organism by means of ultrasound, wherein :
- the process is performed by mechanical treatment using a ball mill equipped with glass balls with a ball filling rate of the mill ranging from 50% to 80% volume/volume, at a temperature ranging from 18 to 40°C, the mean diameter of the balls (d_{GM}) ranges from 1.5 × 10⁻³ to 2.5 × 10⁻³ m and the stirring speed at the blade end (v) ranges from 5 to 20 m.sec⁻¹,
or
- the process is performed by physical treatment of said organism by means of ultrasound according to the following conditions :
- the energy ranges from 500 to 5000 J,
- the power ranges from 200 W to 400 W, and
- the feed flow rate of the reactor ranges from 0.150 l/min to 3 l/min.

2. The process as claimed in claim 1, the mechanical treatment in a ball mill being carried out for a period ranging from 2 to 50 minutes, preferably from 10 to 40 minutes.

3. The process as claimed in one of claims 1 and 2, the ball filling rate of the mill ranging from 60% to 80% and preferably from 65% to 80% volume/volume.

4. The process as claimed in claim 1, the ultrasound treatment being carried out for a period ranging from 5 to 30 seconds.

5. The process as claimed in one of claims 1 to 4, the mechanical or physical treatment step being followed by a step of measuring the amount of membrane polysaccharides extracted.

6. The process as claimed in one of claims 1 to 5, wherein at least one treatment step is followed with a step of measuring the amount of membrane polysaccharides extracted.

7. The process as claimed in one of claims 1 to 8, wherein the organism is selected from: the division Rhodophyta and more specifically from the genera Porphyridium and Rhodella, Spirulina and Dunaliella, and preferably from the following strains: *Porphyridium cruentum, Arthospira platensis, Botryococcus braunii, Cryptecodinium conhii, Chlorella autotrophica, Navicula incerta* and *Rhodosorus marinus.*

8. The process as claimed in one of claims 1 to 9, comprising a step consisting in extracting the soluble polysaccharide fraction from said medium.

9. The process as claimed in one of claims 1 to 13, the membrane polysaccharide extraction step being followed by a step of purifying said polysaccharides.
